# EUROPEAN PATENT SPECIFICATION

(11) **EP 1 176 925 B1**
(45) Date of publication and mention of the grant of the patent: **13.04.2005**
(21) Application number: 00927680.9
(22) Date of filing: 08.05.2000
(51) Int. Cl.: A61F 2/01, A61M 25/01

(54) **A FILTER ELEMENT WITH RETRACTABLE GUIDEWIRE TIP**
FILTERELEMENT MIT ZURÜCKZIEHBARER FÜHRUNGSDRAHTSPITZE
ELEMENT DE FILTRE COMPORTANT UNE POINTE DE FIL DE GUIDAGE RETRACTABLE

(30) Priority: 07.05.1999 WO PCT/IE99/00039
(43) Date of publication of application: 06.02.2002
(73) Proprietor: Salviac Limited, Dublin 2 (IE)
(72) Inventor: BRADY, Eamon, Elphin, County Roscommon (IE)
(74) Representative: O'Brien, John Augustine
(86) International application number: PCT/IE2000/000057
(87) International publication number: WO 2000/067671

(56) References cited:
- EP-A- 0 791 340
- WO-A-98/33443
- US-A- 5 823 992

## Description

This invention relates to a filter element for a transcatheter embolic protection device.

### Introduction

The invention is particularly concerned with filter elements for transcatheter embolic protection devices of the type described in our WO-A-9923976. One type of such embolic filter essentially comprises a filter body mounted on an associated collapsible support frame which can be collapsed against the guidewire by means of a catheter for deployment of the filter through a patient's vascular system. Upon retraction of the catheter the support frame and filter body expand outwardly from the guidewire across a blood vessel within which the filter is positioned to filter blood flowing through the blood vessel.

One problem with the filter device is that there is a guidewire tip on the distal end which is required for guiding the filter into a desired position. The guidewire tip needs to be relatively long to provide a smooth tip transition. However, the guidewire distal tip may interfere with the optimal placement of the filter element.

The present invention is directed towards overcoming this problem.

Document EP-A-0 791 340 discloses an apparatus according to the preamble of claim 1.

### Statements of Invention

The invention is defined in claim 1. Any embodiment which is in contradiction to the subject-matter of claim 1 is not part of the invention.

### Brief Description of the Drawings

The invention will be more clearly understood by the following description of some of the embodiments thereof, given by way of example only, with reference to the accompanying drawings, in which: -
Fig. 1 is partially sectioned elevational view of an embolic protection device;
Fig. 2 is a schematic sectional elevational view of the embolic protection device of Fig. 1;
Fig. 3 is a detail sectional view of a portion of the device of Fig. 1;
Fig. 4 is a longitudinal cross sectional view of the device of Fig. 1;
Fig. 5 is a cross sectional view of a distal end of the device of Fig. 1;
Fig. 6 is a view on the line A-A in Fig. 4;
Fig. 7 is a perspective view of a filter body of the device of Figs. 1 to 6;
Fig. 8 is a side elevational view of the filter body of Fig. 7;
Fig. 9 is a view on a proximal end of the filter body;
Fig. 10 is a perspective view of a support frame of the device of Figs. 1 to 6;
Fig. 11 is a side elevational view of the support frame;
Fig. 12 is a perspective view illustrating the manufacture of the support frame;
Fig. 13 is a view of the support frame and filter body assembly;
Fig. 14 is a side partially cross sectional view of a filter body and guidewire according to one embodiment of the invention in one position of use;
Fig. 15 is a side view similar to Fig. 14 in another position of use;
Fig. 16 is a side, partially cross sectional view of a filter body and guidewire according to another embodiment of the invention in one position of use;
Fig. 17 is a side view similar to Fig. 16 in another position of use;
Fig. 18 is a side partially cross sectional view of a filter body and guidewire according to a further embodiment of the invention in one position of use;
Figs. 19 and 20 are side views similar to Fig. 18 in other positions of use; and
Figs. 21 to 23 are side, partially cross sectional views of a filter body and guidewire according to a further embodiment of the invention in different positions of use.

### Detailed Description

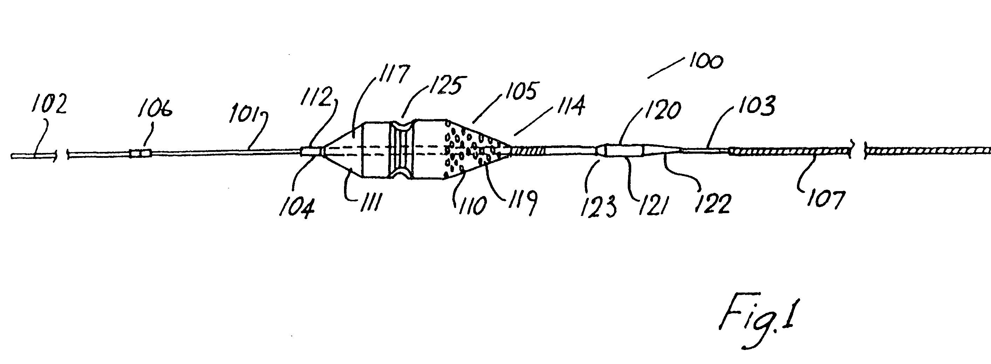

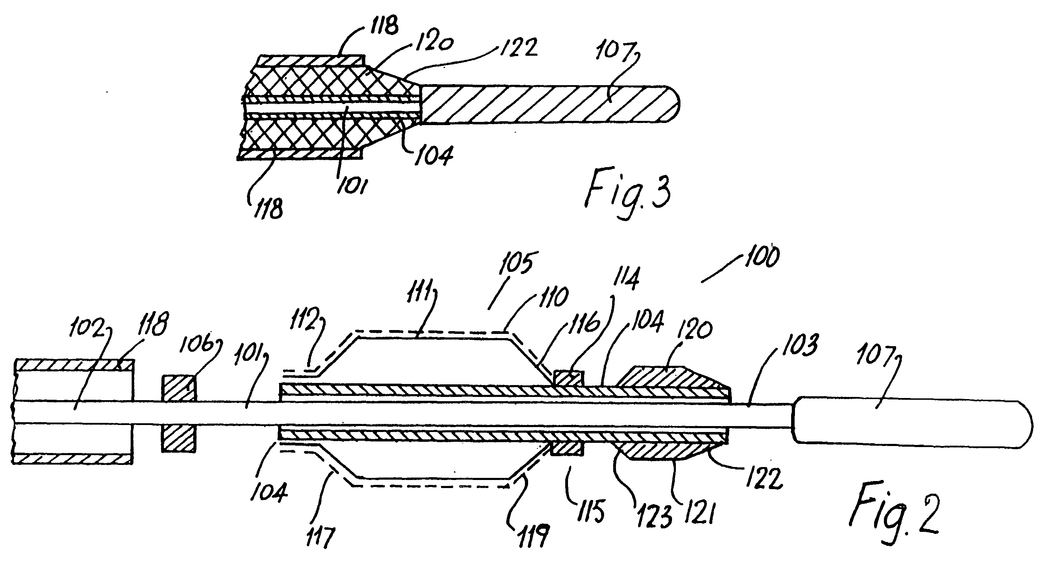

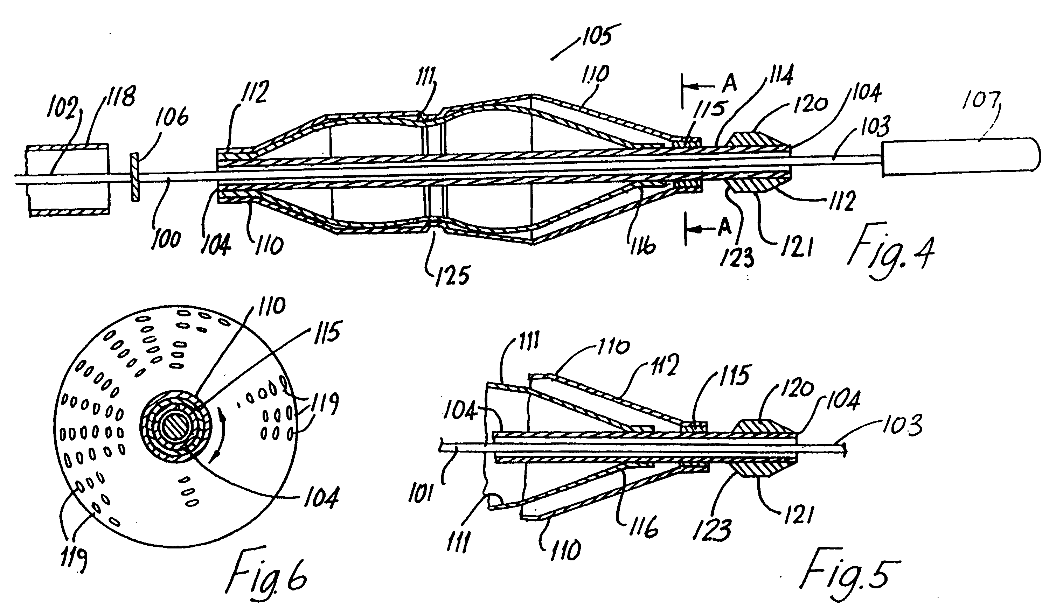

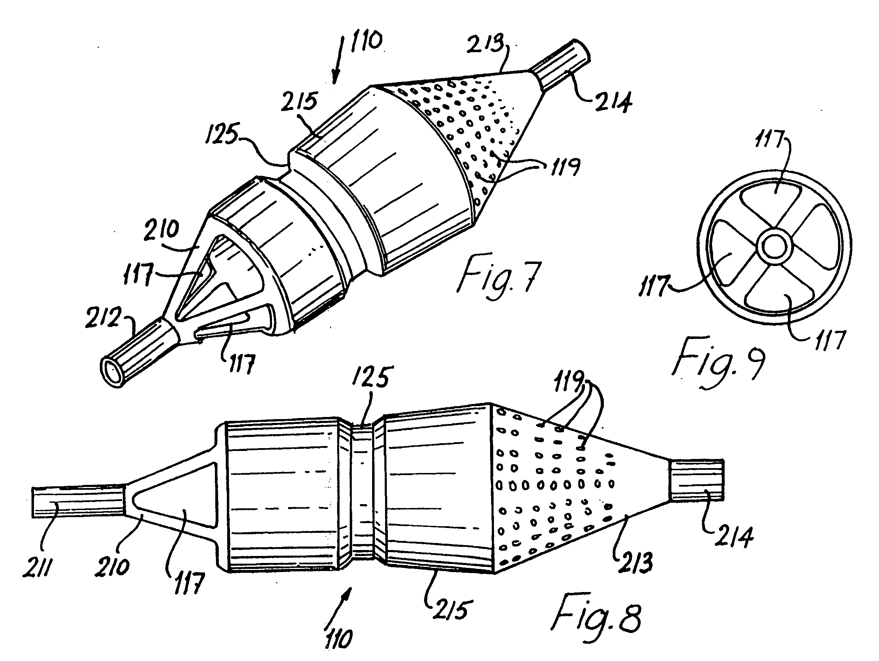

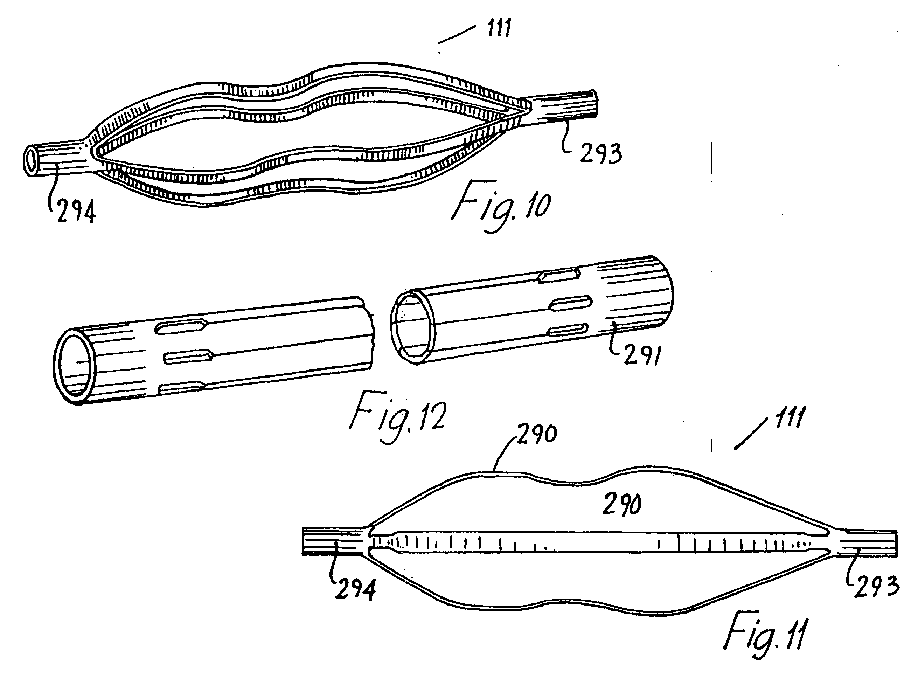

Referring to Figs. 1 to 13 there is illustrated an embolic protection device as described in our WO-A-9923976 indicated generally by the reference number 100. The device 100 has a guidewire 101 with a proximal end 102 and a distal end 103. A tubular sleeve 104 is slidably mounted on the guidewire 101. A collapsible filter 105 is mounted on the sleeve 104, the filter 105 being movable between a collapsed stored position against the sleeve 104 and an expanded position as shown in the drawings extended outwardly of the sleeve 104 for deployment in a blood vessel.

The sleeve 104 is slidable on the guidewire 101 between a pair of spaced-apart end stops, namely an inner stop 106 and an outer stop which in this case is formed by a spring tip 107 at the distal end 103 of the guidewire 101.

The filter 105 comprises a filter body 110 mounted over a collapsible support frame 111. The filter body 110 is mounted to the sleeve 104 at each end, the body 110 being rigidly attached to a proximal end 112 of the sleeve 104 and the body 110 being attached to a collar 115 which is slidable along a distal end 114 of the sleeve 104. Thus the distal end of the body 110 is longitudinally slidable along the sleeve 104. The support frame 111 is also fixed at the proximal end 112 of the sleeve 104. A distal end 116 of the support frame 111 is not attached to the sleeve 104 and is thus also free to move longitudinally along the sleeve 104 to facilitate collapsing the support frame 111 against the sleeve 104. The support frame 111 is such that it is naturally expanded as shown in the drawings and can be collapsed inwardly against the sleeve 104 for loading in a catheter 118 or the like.

The filter body 105 has large proximal inlet openings 117 and small distal outlet openings 119. The proximal inlet openings 117 allow blood and embolic material to enter the filter body, while, the distal outlet openings 119 allow through passage of blood but retain undesired embolic material within the filter body.

An olive guide 120 is mounted at a distal end of the sleeve 104 and has a cylindrical central portion 121 with tapered ends 122, 123. The distal end 122 may be an arrowhead configuration for smooth transition between the catheter and olive surfaces. The support frame 111 is shaped to provide a circumferential groove 125 in the filter body 110. If the filter is too large for a vessel, the body may crease and this groove 125 ensures any crease does not propagate along the filter.

Enlarged openings are provided at a proximal end of the filter body 110 to allow ingress of blood and embolic material into an interior of the body 110.

In use, the filter 105 is mounted in a collapsed state within a distal end of the catheter 118 and delivered to a deployment site. When the filter is correctly positioned the catheter 118 is retracted allowing the support frame 111 to expand expanding the filter body 110 across the vessel in which the filter is mounted. Blood and emboli can enter the enlarged openings at a proximal end of the filter body 110. The blood will pass through the filter body, however, the openings or pores in the filter body are sized so as to retain the embolic material. After use, a retrieval catheter 18 is delivered along the guidewire 101 and slid over the filter 105 engaging the proximal inlet end 112 first to close the openings and then gradually collapsing the filter body against the sleeve 104 as the catheter 118 advances over the filter 105. Once the filter 105 is fully loaded in the catheter 118, it can then be withdrawn.

It will be noted that a proximal end of the filter is fixed and a distal end of the filter is longitudinally movable along the sleeve to facilitate collapsing of the filter body.

Further, the catheter engages the proximal end of the filter body first thus closing the filter body inlet and preventing escape of embolic material from the filter body as the filter body is being collapsed.

The outer filter body 110 is preferably of a resilient biocompatible elastomeric material. The material may be a polyurethane based material. There are a series of commercially available polyurethane materials that may be suitable. These are typically based on polyether or polycarbonate or silicone macroglycols together with diisocyanate and a diol or diamine or alkanolamine or water chain extender. Examples of these are described in EP-A-461,375 and US 5,621, 065. In addition, polyurethane elastomers manufactured from polycarbonate polyols as described in US 5,254,622 (Szycher) are also suitable.

The filter material may also be a biostable polycarbonate urethane article an example of which may be prepared by reaction of an isocyanate, a chain extender and a polycarbonate copolymer polyol of alkyl carbonates. This material is described in our WO-A-9924084. The filter material may be manufactured from a block and cut into a desired shape. However the filter is preferably formed by dipping a rod of desired geometry into a solution of the material which coats the rod. The rod is then dissolved. The final geometry of the filter may be determined in the dipping step or the final geometry may be achieved in a finishing operation. Typically the finishing operations involve processes such as mechanical machining operations, laser machining or chemical machining.

The filter body is of hollow construction and is formed as described above by dipping a rod in a solution of polymeric material to coat the rod. The rod is then dissolved, leaving a hollow body polymeric material. The rod may be of an acrylic material which is dissolved by a suitable solvent such as acetone.

The polymeric body thus formed is machined to the shape illustrated in Figs. I to 13. The final machined filter body comprises an inlet or proximal portion 210 with a proximal neck 212, and outlet or distal portion 213 with a distal neck 214, and an intermediate portion 215 between the proximal and distal portions.

The inlet holes 117 are provided in the proximal portion 210 which allow the blood and embolic material to flow into the filter body. In this case the proximal portion 210 is of generally conical shape to maximise the hole size.

The intermediate portion 215 is also hollow and in this case is of generally cylindrical construction. This is important in ensuring more than simple point contact with the surrounding blood vessel. The cylindrical structure allows the filter body to come into soft contact with the blood vessel to avoid damaging the vessel wall.

The intermediate portion 215 is provided with a radial stiffening means, in this case in the form of a radial strengthening ring or rim 220. The ring 220 provides localised stiffening of the filter body without stiffening the material in contact with the vessel. Such an arrangement provides appropriate structural strength so that line apposition of the filter body to the vessel wall is achieved. It is expected that other geometries of stiffening means will achieve a similar result.

The tubular intermediate portion 215 is also important in maintaining the stability of the filter body in situ to retain captured emboli and to ensure that flow around the filter is minimised. For optimum stability we have found that the ratio of the axial length of the intermediate portion 215 of the filter body to the diameter of the intermediate portion 215 is preferably at least 0.5 and ideally greater than 1.0.

The collapsible support frame 111 has four foldable arms 290 which are collapsed for deployment and upon release extend outwardly to expand the filter body 110.

The support frame 111 can be manufactured from a range of metallic or polymeric components such as a shape memory alloy like nitinol or a shape memory polymer or a shaped stainless steel or metal with similar properties that will recover from the deformation sufficiently to initiate opening of the filter body 110.

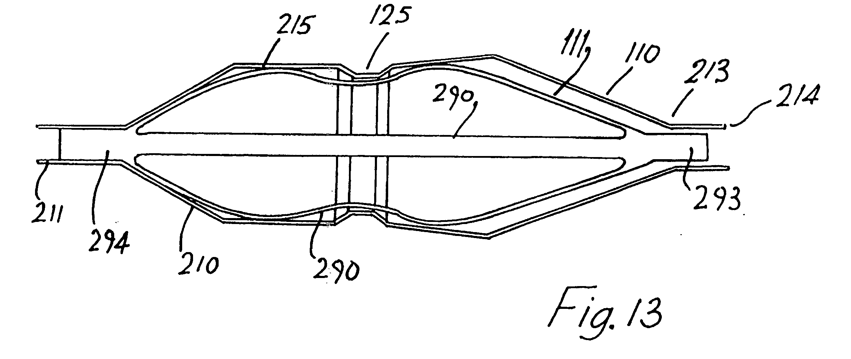

The support frame may be formed as illustrated in Fig. 12 by machining slots in a tube 291 of shape memory alloy such as nitinol. On machining, the unslotted distal end of the tube forms a distal collar 293 and the unslotted proximal end of the tube forms a proximal collar 294. In use, the distal collar 293 is slidably moveable along the tubular sleeve 104 which in turn is slidably mounted on the guidewire 101 for deployment and retrieval. The proximal collar 294 is fixed relative to the tubular sleeve 104.

To load the filter the sub assembly of the support frame and filter body is pulled back into the catheter 118 to engage the distal stop 107. The support arms 290 are hinged inwardly and the distal collar 293 moves forward along the tubular sleeve 104. As the support arms 290 enter the catheter 118 the filter body 110 stretches as the filter body collar 115 slides along the tubular sleeve 104 proximal to the olive 120. On deployment, the catheter 118 is retracted proximally along the guidewire 101 initially bringing the collapsed filter assembly with it until it engages the proximal stop 106. The catheter sleeve then begins to pull off the filter, freeing the support arms 290 to initiate opening of the filter body to appose the vessel wall.

For retrieval, a retrieval catheter is introduced by sliding it over the guidewire 101 until it is positioned at the proximal end of the filter body and support frame. Pulling the guidewire 101 will initially engage the distal stop 107 with the filter element and begin to pull it into the retrieval catheter. The initial travel into the delivery catheter acts to close the proximal openings of the filter element, thus entrapping the embolic load. As the filter continues to be pulled back the filter body and the support frame are enveloped in the retrieval catheter. The collapsed filter may then be removed from the patient.

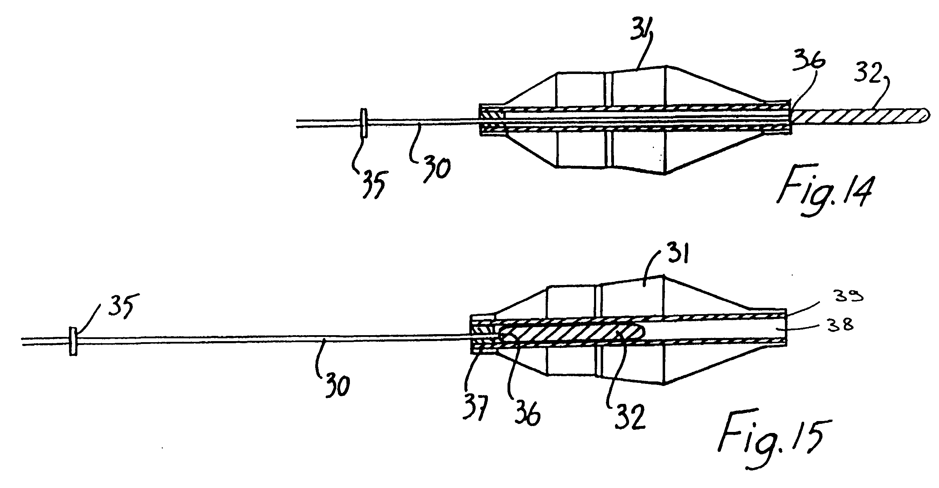

Referring to Figs. 14 and 15 there is illustrated a medical guidewire assembly according to one embodiment of the invention. A filter 31 is mounted on a guidewire 30 and projecting from the distal end of the guidewire 30 is a guidewire tip 32. The guidewire tip 32 is slidable in a bore 38 in a sleeve 39 of the filter 31. When the filter 31 is being manoeuvred into place the guidewire tip 32 facilitates the manoeuvring of the filter device. By advancing and retracting the tip 32 relative to the filter assembly 31 it is possible to manoeuvre the guidewire tip 32 around various portions of the anatomy, for example, where it is particularly tortuous, or where the guidewire tip 32 has to cross lesions. The tip 32 can be partially retracted to give a stiffer tip, or can be fully retracted in the deployment position, Fig. 15.

The guidewire 30 is slidable between a proximal guidewire limiting element 35 on the guidewire 30 and a filter limiting element 37 provided at a proximal end of the filter 31. A stop defined by a shoulder 36 of the tip 32 is engagable against the limiting element 37.

The proximal limiting element 35 and the filter limiting element 37 are of a relatively stiff material, such that upon engagement of the filter 31 with the proximal limiting element 35, or the shoulder 36 with the filter limiting element 37, the limiting elements 35, 37 do not deform. In this way the movement of the filter 31 relative to the guidewire tip 32 is accurately controlled.

One or both of the limiting elements 35, 37 may be of a compliant material. This feature will assist in ensuring that the flexibility of the filter is not affected by the limiting elements.

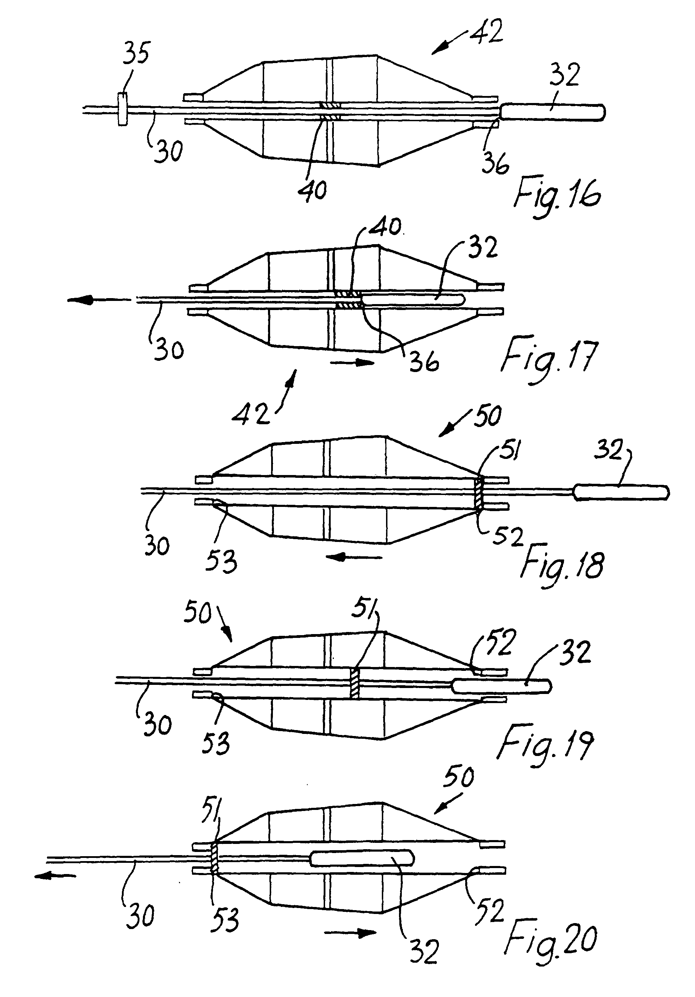

Referring to Figs. 16 and 17 there is illustrated a medical guidewire assembly including a filter 42, which is similar to the filter 31 of Figs. 14 and 15, and the same reference numerals are used to denote similar elements in Figs. 16 and 17. In this case the guidewire 30 is slidable between a proximal limiting element 35 on the guidewire 30 and a filter limiting element 40 positioned intermediate the proximal and distal ends of the filter 42. A distal stop defined by a shoulder 36 of the tip 32 is engagable against the filter limiting element 40.

Referring to Figs. 18 to 20 there is illustrated a medical guidewire assembly including a filter 50, which is similar to filters 31 and 42 of Figs. 14 to 17, and the same reference numerals are used to denote similar elements in Figs. 18 to 20. In this arrangement a guidewire limiting element 51 is rigidly fixed to the guidewire 30 proximal of the tip 32, the filter 50 being mounted on the guidewire 30 so that the limiting element 51 is intermediate the proximal and distal ends of the filter 50. The guidewire 30 is slidable between a distal limiting element defined by a proximal shoulder 53 of the filter 50 which is engagable against the guidewire limiting element 51, and a proximal limiting element defined by a distal shoulder 52 of the filter 50 which is engagable against the-guidewire limiting element 51. In this arrangement there is no obstruction to advancement of another medical device over the guidewire 30 to approach the filter 50 from the proximal direction.

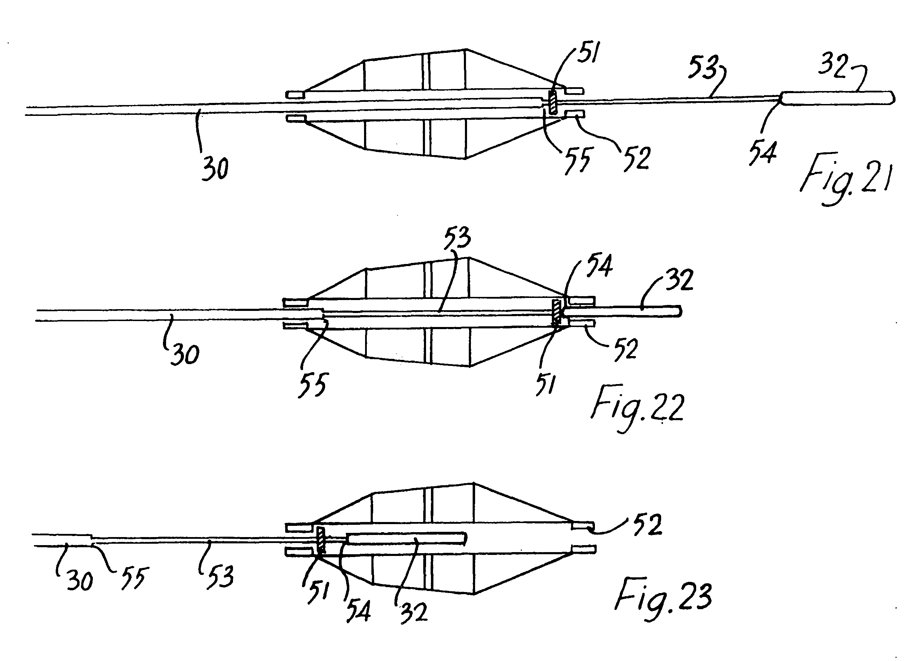

Referring to Figs. 21 to 23 in an alternative embodiment of the invention, the guidewire limiting element 51 is slidably mounted within a recess 53 provided on the guidewire 30, the movement of the limiting element 51 relative to the guidewire 30 being limited between a proximal stop provided by a shoulder 55 of the recess and a distal stop provided by a shoulder 54 provided by the guidewire tip 32. This arrangement provides an even greater degree of freedom for movement of the guidewire 30 relative to the filter.

The filter may be placed over or beyond the distal guidewire tip. Thus, the invention facilitates the optimal placement of a filter device in the limited vasculature space available.

Other medical devices may be advanced over the guidewire to approach the filter from the proximal direction without obstruction. Such devices may be for use in performing angioplasty procedures, stenting and the like. Ready access is also provided to perform emergency procedures such as snaring of a medical device or part, and lysis for treatment of a blood clot.

The invention is not limited to the embodiments hereinbefore described which may be varied in both construction and detail.

## Claims

1. An embolic protection device comprising:
a guidewire having a flexible tip at a distal end of the guidewire;
a collapsible filter element;
the filter element being movable between a collapsed stored position against the guidewire for movement through the vascular system, and an expanded position for occluding a blood vessel such that blood passing through the blood vessel is delivered through the filter element;
the filter element comprising a collapsible filter body having an inlet end and an outlet end;
the inlet end of the filter body having one or more inlet openings sized to allow blood and embolic material enter the filter body;
the outlet end of the.filter body having a plurality of outlet openings sized to allow through passage of blood but to retain undesired embolic material within the filter body;
the filter element being mounted near the distal end of the guidewire proximally of the tip, the filter element being movable, independently of the movement between the collapsed stored position and the expanded position, relative to the tip for adjustment of the amount of the tip extending distally of the filter element; **characterized by**
means adapted to limit the movement of the filter element, independent of the movement between the collapsed stored position and the expanded position, relative to the tip.

2. An embolic protection device as claimed in claim 1 wherein the means to limit the movement of the medical device comprises one or more stiff limiting elements.

3. An embolic protection device as claimed in claim 2 wherein at least one limiting element is provided on the guidewire.

4. An embolic protection device as claimed in claim 3 wherein the limiting element is fixedly mounted to the guidewire.

5. An embolic protection device as claimed in claim 3 wherein the limiting element is slidably mounted on the guidewire.

6. An embolic protection device as claimed in claim 5 including stop means to limit slidable movement of the limiting element relative to the guidewire.

7. An embolic protection device as claimed in claim 6 wherein the stop means to limit slidable movement of the limiting element comprises a pair of stops spaced axially apart along the guidewire.

8. An embolic protection device as claimed in claim 7 wherein the stops are provided by abutment surfaces formed in the guidewire.

9. An embolic protection device as claimed in any of claims 2 to 8 wherein at least one limiting element is mounted to the filter element.

10. An embolic protection device as claimed in claim 9 wherein the limiting element is mounted to the filter element at the proximal end of the filter element.

11. An embolic protection device as claimed in claim 9 wherein the limiting element is mounted intermediate proximal and distal ends of the filter element.

12. An embolic protection device as claimed in any of claims 2 to 11 wherein at least one limiting element is stiff relative to the guidewire.

13. An embolic protection device as claimed in any of claims 2 to 12 wherein at least one limiting element is compliant relative to the guidewire.

14. An embolic protection device as claimed in any preceding claim wherein the filter element and the tip are slidable relative to each other.

15. An embolic protection device as claimed in any preceding claim wherein the filter element has a receiver slot for reception of at least portion of the tip.

16. An embolic protection device as claimed in claim 15 wherein the tip is fully retractable within the receiver slot.

17. An embolic protection device as claimed in any preceding claim wherein the filter element comprises a tubular sleeve which is slidably mounted on the guidewire adjacent the distal end of the guidewire, the sleeve having a bore through which the guidewire passes, said bore forming a receiver slot for reception of the flexible tip of the guidewire which is at least partially retractable within the bore of the sleeve.

18. An embolic protection device as claimed in claim 17 wherein the tip is fully retractable within the bore of the sleeve.

19. An embolic protection device as claimed in claim 17 or 18 wherein a guidewire limiting element is mounted to the guidewire proximal of the filter element and a filter limiting element is mounted to the filter element within the bore of the sleeve, the guidewire being movable relative to the filter element between the first and second limiting elements.

20. An embolic protection device as claimed in claim 19 wherein the guidewire has an abutment which is engagable with the filter limiting element when the guidewire tip is retracted.

21. An embolic protection device as claimed in claim 20 wherein the abutment is provided by a shoulder of the tip.

22. An embolic protection device as claimed in any of claims 19 to 21 wherein the filter limiting element is provided at a proximal end of the filter element.

23. An embolic protection device as claimed in any of claims 19 to 21 wherein the filter limiting element is provided intermediate proximal and distal ends of the filter element.

24. An embolic protection device as claimed in claim 17 or 18 wherein a guidewire limiting element is mounted to the guidewire intermediate proximal and distal ends of the filter element and the filter element has a proximal filter limiting element and a distal filter limiting element, the guidewire limiting element being movable with the guidewire between the proximal and distal filter limiting elements.

25. An embolic protection device as claimed in claim 24 wherein the guidewire tip is retractable proximally of the distal filter limiting element.

26. An embolic protection device as claimed in claim 24 wherein the guidewire limiting element is movable on the guidewire.

27. An embolic protection device as claimed in claim 26 including stop means to limit slidable movement of the guidewire limiting element relative to the guidewire.

28. An embolic protection device as claimed in claim 27 wherein the stop means comprises a pair of stops spaced axially apart along the guidewire.

29. An embolic protection device as claimed in claim 28 wherein the stops are provided by abutment surfaces formed in the guidewire.

30. An embolic protection device as claimed in any of claims 27 to 29 wherein the guidewire has a recessed portion of reduced diameter on which the guidewire limiting element is mounted.

## Patentansprüche

1. Embolieschutzvorrichtung, die Folgendes umfasst:
einen Führungsdraht mit einer elastischen Spitze an einem distalen Ende des Führungsdrahtes;
ein zusammenklappbares Filterelement;
wobei das Filterelement zwischen einer zusammengeklappten gelagerten Position gegen den Führungsdraht zur Bewegung durch das Gefäßsystem und einer ausgedehnten Position zum Okkludieren eines Blutgefäßes beweglich ist, so dass Blut, das durch das Blutgefäß strömt, durch das Filterelement geführt wird;
das Filterelement einen zusammenklappbaren Filterkörper mit einem Einlassende und einem Auslassende umfasst;
das Einlassende des Filterkörpers eine oder mehrere Einlassöffnungen aufweist, die so bemessen sind, dass sie das Eindringen von Blut und Emboliematerial in den Filterkörper ermöglichen;
das Auslassende des Filterkörpers eine Vielzahl von Auslassöffnungen aufweist, die so bemessen sind, dass sie den Durchstrom von Blut ermöglichen, jedoch unerwünschtes Emboliematerial in dem Filterkörper zurückhalten;
das Filterelement in der Nähe des distalen Endes des Führungsdrahtes proximal zur Spitze befestigt ist und das Filterelement unabhängig von der Bewegung zwischen der zusammengefalteten gelagerten Position und der ausgedehnten Position im Verhältnis zu der Spitze beweglich ist, um das Ausmaß der Ausdehnung der Spitze distal zum Filterelement einzustellen; **gekennzeichnet durch**
ein Mittel, das dafür ausgelegt ist, die Bewegung des Filterelements unabhängig von der Bewegung zwischen der zusammengefalteten gelagerten Position und der ausgedehnten Position im Verhältnis zu der Spitze zu beschränken.

2. Embolieschutzvorrichtung nach Anspruch 1, wobei das Mittel zur Beschränkung der Bewegung der medizinischen Vorrichtung ein oder mehrere steife Begrenzungselemente umfasst.

3. Embolieschutzvorrichtung nach Anspruch 2, wobei mindestens ein Begrenzungselement auf dem Führungsdraht bereitgestellt ist.

4. Embolieschutzvorrichtung nach Anspruch 3, wobei das Begrenzungselement fest am Führungsdraht befestigt ist.

5. Embolieschutzvorrichtung nach Anspruch 3, wobei das Begrenzungselement gleitfähig auf dem Führungsdraht befestigt ist.

6. Embolieschutzvorrichtung nach Anspruch 5, die ein Anschlagmittel zur Beschränkung der Gleitbewegung des Begrenzungselements im Verhältnis zum Führungsdraht umfasst.

7. Embolieschutzvorrichtung nach Anspruch 6, wobei das Anschlagmittel zur Beschränkung der Gleitbewegung des Begrenzungselements ein Paar Anschläge umfasst, die axial entlang des Führungsdrahts beabstandet sind.

8. Embolieschutzvorrichtung nach Anspruch 7, wobei die Anschläge durch Anschlagflächen bereitgestellt sind, die in dem Führungsdraht gebildet sind.

9. Embolieschutzvorrichtung nach einem der Ansprüche 2 bis 8, wobei mindestens ein Begrenzungselement am Filterelement befestigt ist.

10. Embolieschutzvorrichtung nach Anspruch 9, wobei das Begrenzungselement am Filterelement am proximalen Ende des Filterelements befestigt ist.

11. Embolieschutzvorrichtung nach Anspruch 9, wobei das Begrenzungselement zwischen dem proximalen und dem distalen Ende des Filterelements befestigt ist.

12. Embolieschutzvorrichtung nach einem der Ansprüche 2 bis 11, wobei mindestens ein Begrenzungselement im Verhältnis zum Führungsdraht steif ist.

13. Embolieschutzvorrichtung nach einem der Ansprüche 2 bis 12, wobei mindestens ein Begrenzungselement im Verhältnis zum Führungsdraht nachgiebig ist.

14. Embolieschutzvorrichtung nach einem der vorhergehenden Ansprüche, wobei das Filterelement und die Spitze im Verhältnis zueinander gleitfähig sind.

15. Embolieschutzvorrichtung nach einem der vorhergehenden Ansprüche, wobei das Filterelement einen Aufnahmeschlitz zur Aufnahme mindestens eines Abschnitts der Spitze aufweist.

16. Embolieschutzvorrichtung nach Anspruch 15, wobei die Spitze in dem Aufnahmeschlitz vollständig zurückgezogen werden kann.

17. Embolieschutzvorrichtung nach einem der vorhergehenden Ansprüche, wobei das Filterelement eine röhrenförmige Hülle umfasst, die gleitfähig auf dem Führungsdraht benachbart zum distalen Ende des Führungsdrahts befestigt ist, die Hülle eine Bohrung aufweist, durch die der Führungsdraht läuft, und die Bohrung einen Aufnahmeschlitz zur Aufnahme der elastischen Spitze des Führungsdrahtes bildet, die mindestens teilweise in der Bohrung der Hülle zurückgezogen werden kann.

18. Embolieschutzvorrichtung nach Anspruch 17, wobei die Spitze in der Bohrung der Hülle vollständig zurückgezogen werden kann.

19. Embolieschutzvorrichtung nach Anspruch 17 oder 18, wobei ein Führungsdrahtbegrenzungselement proximal zum Filterelement am Führungsdraht befestigt ist und ein Filterbegrenzungselement in der Bohrung der Hülle am Filterelement befestigt ist, wobei der Führungsdraht im Verhältnis zum Filterelement zwischen dem ersten und dem zweiten Begrenzungselement beweglich ist.

20. Embolieschutzvorrichtung nach Anspruch 19, wobei der Führungsdraht ein Anschlagelement aufweist, das in das Filterbegrenzungselement eingreifen kann, wenn die Führungsdrahtspitze zurückgezogen ist.

21. Embolieschutzvorrichtung nach Anspruch 20, wobei das Anschlagelement durch eine Schulter der Spitze bereitgestellt wird.

22. Embolieschutzvorrichtung nach einem der Ansprüche 19 bis 21, wobei das Filterbegrenzungselement an einem proximalen Ende des Filterelements bereitgestellt ist.

23. Embolieschutzvorrichtung nach einem der Ansprüche 19 bis 21, wobei das Filterbegrenzungselement zwischen dem proximalen und dem distalen Ende des Filterelements bereitgestellt ist.

24. Embolieschutzvorrichtung nach einem der Ansprüche 17 oder 18, wobei ein Führungsdrahtbegrenzungselement zwischen dem proximalen und dem distalen Ende des Filterelements am Führungsdraht befestigt ist und das Filterelement ein proximales Filterbegrenzungselement und ein distales Filterbegrenzungselement aufweist, wobei das Führungsdrahtbegrenzungselement mit dem Führungsdraht zwischen dem proximalen und dem distalen Filterbegrenzungselement beweglich ist.

25. Embolieschutzvorrichtung nach Anspruch 24, wobei die Führungsdrahtspitze proximal zum distalen Filterbegrenzungselement zurückgezogen werden kann.

26. Embolieschutzvorrichtung nach Anspruch 24, wobei das Führungsdrahtbegrenzungselement auf dem Führungsdraht beweglich ist.

27. Embolieschutzvorrichtung nach Anspruch 26, die ein Anschlagmittel zur Beschränkung der Gleitbewegung des Führungsdrahtbegrenzungselements im Verhältnis zu dem Führungsdraht umfasst.

28. Embolieschutzvorrichtung nach Anspruch 27, wobei das Anschlagmittel ein Paar Anschläge umfasst, die axial entlang des Führungsdrahtes beabstandet sind.

29. Embolieschutzvorrichtung nach Anspruch 28, wobei die Anschläge durch Anschlagoberflächen bereitgestellt sind, die in dem Führungsdraht gebildet sind.

30. Embolieschutzvorrichtung nach einem der Ansprüche 27 bis 29, wobei der Führungsdraht einen vertieften Abschnitt mit verringertem Durchmesser aufweist, auf dem das Führungsdrahtbegrenzungselement befestigt ist.

## Revendications

1. Dispositif de protection embolique comprenant :
un fil-guide ayant un bout flexible à l'extrémité distale du fil-guide ;
un élément filtrant repliable ;
cet élément filtrant pouvant être bougé entre une position repliée de rangement contre le fil-guide pour le mouvement à travers le système vasculaire, et une position déployée pour occlure un vaisseau sanguin de façon à ce que le sang traversant ce vaisseau sanguin soit transporté à travers l'élément filtrant ;
cet élément filtrant comprenant un corps de filtre repliable ayant une extrémité d'entrée et une extrémité de sortie ;
l'extrémité d'entrée du corps du filtre ayant une ou plusieurs ouvertures d'entrée dimensionnées pour permettre au sang et à le matériau embolique d'entrer dans le corps du filtre ;
l'extrémité de sortie du corps du filtre ayant une pluralité d'ouvertures de sortie dimensionnées pour permettre le passage du sang à travers elles mais pour retenir le matériau embolique indésirable à l'intérieur du corps du filtre ;
l'élément filtrant étant monté près de l'extrémité distale du fil-guide, proximalement au bout, l'élément filtrant pouvant être bougé, indépendamment du mouvement entre la position répliée de rangement et de la position déployée, relativement au bout pour régler la quantité dont le bout fait saillie distalement de l'élément filtrant ; **caractérisé par** un moyen adapté pour limiter le mouvement de l'élément filtrant, indépendant du mouvement entre la position repliée de rangement et la position déployée, relativement au bout.

2. Dispositif de protection embolique tel que revendiqué dans la revendication 1, dans lequel le moyen pour limiter le mouvement du dispositif médical comprend un ou plusieurs éléments limiteurs rigides.

3. Dispositif de protection embolique tel que revendiqué dans la revendication 2, dans lequel au moins un élément limiteur est prévu sur le fil-guide.

4. Dispositif de protection embolique tel que revendiqué dans la revendication 3, dans lequel l'élément limiteur est monté sur le fil-guide de manière fixe.

5. Dispositif de protection embolique tel que revendiqué dans la revendication 3, dans lequel l'élément limiteur est monté sur le fil-guide de manière à pouvoir glisser.

6. Dispositif de protection embolique tel que revendiqué dans la revendication 5 comprenant un moyen d'arrêt pour limiter le mouvement de glissement de l'élément limiteur relativement au fil-guide.

7. Dispositif de protection embolique tel que revendiqué dans la revendication 6, dans lequel le moyen d'arrêt pour limiter le mouvement de glissement de l'élément limiteur comprend une paire de butées espacées axialement le long du fil-guide.

8. Dispositif de protection embolique tel que revendiqué dans la revendication 7, dans lequel les butées sont fournies par des surfaces d'aboutement formées dans le fil-guide.

9. Dispositif de protection embolique tel que revendiqué dans l'une quelconque des revendications 2 à 8, dans lequel au moins un élément limiteur est monté sur l'élément filtrant.

10. Dispositif de protection embolique tel que revendiqué dans la revendication 9, dans lequel l'élément limiteur est monté sur l'élément filtrant à l'extrémité proximale de l'élément filtrant.

11. Dispositif de protection embolique tel que revendiqué dans la revendication 9, dans lequel l'élément limiteur est monté dans une position intermédiaire aux extrémités proximale et distale de l'élément filtrant.

12. Dispositif de protection embolique tel que revendiqué dans l'une quelconque des revendications 2 à 11, dans lequel au moins un élément limiteur est rigide relativement au fil-guide.

13. Dispositif de protection embolique tel que revendiqué dans l'une quelconque des revendications 2 à 12, dans lequel au moins un élément limiteur est souple relativement au fil-guide.

14. Dispositif de protection embolique tel que revendiqué dans l'une quelconque des revendications précédentes, dans lequel l'élément filtrant et le bout peuvent glisser l'un relativement à l'autre.

15. Dispositif de protection embolique tel que revendiqué dans l'une quelconque des revendications précédentes, dans lequel l'élément filtrant a une fente réceptrice pour la réception d'au moins une portion du bout.

16. Dispositif de protection embolique tel que revendiqué dans la revendication 15, dans lequel le bout est entièrement rétractable à l'intérieur de la fente réceptrice.

17. Dispositif de protection embolique tel que revendiqué dans l'une quelconque des revendications précédentes, dans lequel l'élément filtrant comprend un manchon tubulaire qui est monté sur le fil-guide, de façon à pouvoir glisser, dans une position adjacente à l'extrémité distale du fil-guide, ce manchon ayant un alésage à travers lequel le fil-guide passe, ledit alésage formant une fente réceptrice pour la réception du bout flexible du fil-guide qui est au moins partiellement rétractable à l'intérieur de l'alésage du manchon.

18. Dispositif de protection embolique tel que revendiqué dans la revendication 17, dans lequel le bout est complètement rétractable à l'intérieur de l'alésage du manchon.

19. Dispositif de protection embolique tel que revendiqué dans la revendication 17 ou 18, dans lequel un élément limiteur du fil-guide est monté sur le fil-guide proximalement à l'élément filtrant et un élément limiteur du filtre est monté sur l'élément filtrant à l'intérieur de l'alésage du manchon, le fil-guide pouvant être bougé relativement à l'élément filtrant entre le premier et le deuxième élément limiteur.

20. Dispositif de protection embolique tel que revendiqué dans la revendication 19, dans lequel le fil-guide a un about qui peut être engagé avec l'élément limiteur du filtre lorsque le bout du fil-guide est rétracté.

21. Dispositif de protection embolique tel que revendiqué dans la revendication 20, dans lequel l'about est fourni par un épaulement du bout.

22. Dispositif de protection embolique tel que revendiqué dans l'une quelconque des revendications 19 à 21, dans lequel l'élément limiteur du filtre est prévu à l'extrémité proximale de l'élément filtrant.

23. Dispositif de protection embolique tel que revendiqué dans l'une quelconque des revendications 19 à 21, dans lequel l'élément limiteur du filtre est prévu dans une position intermédiaire aux extrémités proximale et distale de l'élément filtrant.

24. Dispositif de protection embolique tel que revendiqué dans la revendication 17 ou 18, dans lequel un élément limiteur de fil-guide est monté sur le fil-guide dans une position intermédiaire entre les extrémités proximale et distale de l'élément filtrant et l'élément filtrant a un élément limiteur proximal du filtre et un élément limiteur distal du filtre, l'élément limiteur du fil-guide pouvant être bougé avec le fil-guide entre les éléments limiteurs proximal et distal du filtre.

25. Dispositif de protection embolique tel que revendiqué dans la revendication 24, dans lequel le bout du fil-guide est rétractable proximalement à l'élément limiteur distal du filtre.

26. Dispositif de protection embolique tel que revendiqué dans la revendication 24, dans lequel l'élément limiteur du fil-guide peut être bougé sur le fil-guide.

27. Dispositif de protection embolique tel que revendiqué dans la revendication 26, comprenant un moyen d'arrêt pour limiter le mouvement de glissement de l'élément limiteur du fil-guide relativement au fil-guide.

28. Dispositif de protection embolique tel que revendiqué dans la revendication 27, dans lequel le moyen d'arrêt comprend une paire de butées espacées axialement le long du fil-guide.

29. Dispositif de protection embolique tel que revendiqué dans la revendication 28, dans lequel les butées sont fournies par les surfaces d'aboutement formées dans le fil-guide.

30. Dispositif de protection embolique tel que revendiqué dans l'une quelconque des revendications 27 à 29, dans lequel le fil-guide a une portion en retrait de diamètre réduit sur laquelle l'élément limiteur du fil-guide est monté.
